Europäisches Patentamt

European Patent Office

Office européen des brevets

(19)

(11) Veröffentlichungsnummer : **0 077 809**
**B1**

(12) **EUROPÄISCHE PATENTSCHRIFT**

(45) Veröffentlichungstag der Patentschrift :
11.11.87

(51) Int. Cl.⁴ : **A 61 N   1/368**

(21) Anmeldenummer : 82901500.7

(22) Anmeldetag : 04.05.82

(86) Internationale Anmeldenummer :
PCT/DE 82/00105

(87) Internationale Veröffentlichungsnummer :
WO/8203786 (11.11.82 Gazette 82/27)

(54) **HERZSCHRITTMACHER.**

(30) Priorität : 04.05.81 DE 3118094

(43) Veröffentlichungstag der Anmeldung :
04.05.83 Patentblatt 83/18

(45) Bekanntmachung des Hinweises auf die Patenterteilung : 11.11.87 Patentblatt 87/46

(84) Benannte Vertragsstaaten :
DE FR GB NL

(56) Entgegenhaltungen :
FR-A- 2 082 703
FR-A- 2 365 337
GB-A- 2 026 870
US-A- 4 059 116
US-A- 4 060 090

(73) Patentinhaber : BIOTRONIK Mess- und Therapiegeräte GmbH & Co Ingenieurbüro Berlin
Sieversufer 8
D-1000 Berlin 47 (DE)

(72) Erfinder : NETTELHORST, Herwig, Frhr. v.
Pössnecker Strasse 30
D-1000 Berlin 45 (DE)
Erfinder : REXHAUSEN, Hermann
Mellinger Strasse 48
D-3200 Hildesheim (DE)
Erfinder : SCHALDACH, Max
Turnstrasse 5
D-8520 Erlangen (DE)
Erfinder : SCHWEIGGERT; Eugen
Selbitzer Strasse 81 a
D-1000 Berlin 22 (DE)

(74) Vertreter : Christiansen, Henning, Dipl.-Ing.
CHRISTIANSEN & NINNEMANN Dietrich-Schäfer-Weg 21
D-1000 Berlin 41 (DE)

EP 0 077 809 B1

## Beschreibung

Die Erfindung betrifft einen Herzschrittmacher der im Oberbegriff des Anspruchs 1 angegebenen Art.

Es ist aus der GB-A-2 026 870 bei einem künstlichen Herzschrittmacher bekannt, Refraktärzeiten vorzusehen, während der von den Eingangsschaltungen aufgenommene Signale im Atrium und/oder Ventrikel nicht für die direkte Steuerung der Ausgangsmittel zur Abgabe von Stimulationsimpulsen herangezogen werden. Dadurch wird erreicht, daß eine Stimulationsimpulsabgabe zu diesen Zeiten verhindert wird und auch keine unerwünschte Synchronisation der Zeitgebermittel mit zu den betreffenden Zeiten erscheinenden Impulsen stattfindet.

Es ist bei Schrittmachern mit Mitteln zur Signalaufnahme im Atrium und im Ventrikel und veränderbarer Dauer zwischen denjenigen Zeitpunkten, zu denen eine Stimulation im Atrium (gegebenenfalls bei nichtvorhandener Eigenaktivität) stattfindet, nachteilig, wenn die Dauer der Refraktärzeit nicht mit den sich einstellenden physiologischen Verhältnissen übereinstimmt und jeweils zusätzlich individuell festgelegt werden muß.

Fehlerhafte Refraktärzeiten können bei inhibierenden Schrittmachern zu fehlerhafter Synchronisation und bei synchronisierenden Schrittmachern zu Schrittmacher-Parasystolien führen.

Der Erfindung liegt die Aufgabe zugrunde, eine Anordnung anzugeben, welche zur Anpassung der Refraktärzeit des Ventrikels an das Escape-Intervall bzw. die asynchrone Grundrate des Atriums keine zusätzlichen Handhabungen erfordert. Diese Aufgabe ist durch die im Anspruch 1 angegebenen Maßnahmen gelöst.

Besonders vorteilhaft ist dabei, daß auch bei Änderung der für die Signalaufnahme bzw. die Stimulation des Atriums maßgeblichen Zeiten aufgrund von im Herzen selbst aufgenommenen Signalen keine Veränderung der Refraktärzeit durch äußere Mittel vorgenommen werden muß.

Die Erfindung beruht dabei auf der Erkenntnis, daß die Einstellung der Refraktärzeiten für die Signalaufnahmen vom Ventrikel stets mit besonderer Genauigkeit vorgenommen werden muß, da gewisse Abweichungen bei der Festlegung der Refraktärzeit des Atriums zwar hingenommen werden können, da sie zu einem Stimulationszustand mit 2 : 1-Überleitung führen, daß aber eine entsprechende Möglichkeit für den Ventrikel nicht besteht.

Bei bevorzugten Weiterbildungen der Erfindung wird die Errechnung der Refraktärzeit nach der Formel :

$$T_{refVaut} = (1/2 - 1/8) \cdot T_A$$

vorgenommen, welche sich insbesondere mit binären Schaltmitteln leicht ausführen läßt.

Die Berechnung der Refraktärzeit erfolgt selbsttätig im Zusammenhang mit jeder Veränderung der die Grundrate im Atrium $(T_A)$ bestimmenden Signalzustände. Bei der Umprogrammierung des Atrium-Escape-Intervalls ist daher bei programmierbaren Schrittmachern keine Veränderung der Refraktärzeit des Ventrikels durch zusätzliche aüßere Programmiermaßnahmen erforderlich.

Die Erfindung basiert auf dem Bestreben, eine universelle Schrittmacherstruktur anzugeben, welche Fehlermöglichkeiten bekannter Systeme vermeidet, wobei die Realisierung in einem implantierbaren System möglich sein soll. Das System soll eine weitestgehende Auswertung der aufgrund von vom Herzen abgeleiteten Signalen zur Verfügung stehender Informationen ermöglichen, wobei der Einfluß von Störsignalen gering gehalten ist. Durch die physiologisch korrekte Zuordnung aller die Betriebsarten des Schrittmachers beteffenden Umschaltvorgänge wird eine große Patientensicherheit erreicht.

Vorteilhafte Weiterbildungen sind in den Unteransprüchen angegeben und werden einschließlich der Erfindung anhand des in den Figuren dargestellten Ausführungsbeispiels näher erläutert. Es zeigen :

Figur 1 ein Blockschaltbild der den erfindungsgemäßen Schrittmacher bildenden Baugruppen unter besonderer Berücksichtigung der im Betrieb auftretenden logischen Verknüpfungen,

Figur 2 ein Zeitdiagramm zur Erläuterung des Funktionsablaufs bei dem in Fig. 1 dargestellten Schrittmacher,

Figur 3 Einzelheiten einer Anordnung zum Umschalten in einen Hysteresezustand als Teil einer der in Fig. 1 dargestellten Anordnung hinzuzufügenden Baugruppe,

Figur 4a Einzelheiten einer Baugruppe zur automatischen Berechnung der Refraktärzeit des Ventrikels, welche dem Blockschaltbild gemäß Fig. 1 hinzuzufügen ist, sowie

Figur 4b Einzelheiten einer der in Fig. 4a dargestellten Baugruppen.

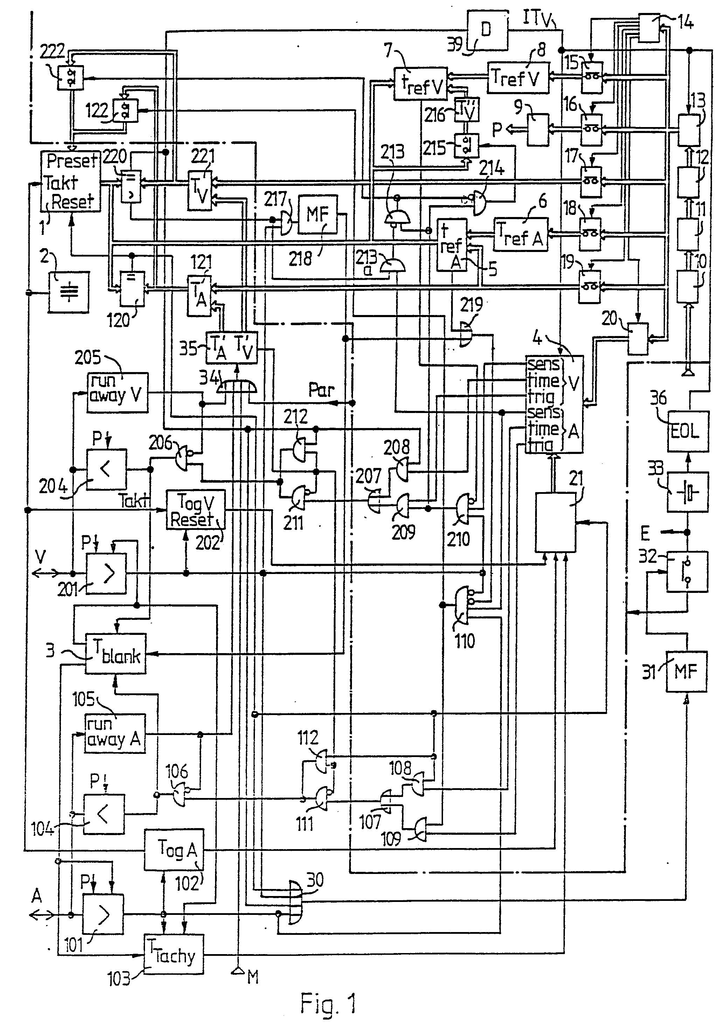

Das in Figur 1 dargestellte in Blockschaltungsform wiedergegebene Prinzipschaltbild des erfindungsgemäßen Herzschrittmachers gibt den Signalfluß zwischen den einzelnen Baugruppen und eine Anzahl von logischen Verknüpfungen wieder. Der verwendeten Schrittmacherstruktur liegt das Konzept eines Schrittmachers zugrunde, das auf grund von Zu- und Abschaltungen von Signalverbindungen in unterschiedlichen Betriebsarten (Modes) benutzt werden kann, wobei die Umschaltung zwischen den einzelnen Betriebsarten (die im Folgenden mit den auf dem Gebiet der Herzschrittmachertechnik eingeführten generischen Codes bezeichnet werden sollen) programmiert und/oder signalabhängig erfolgen kann. In seinen komplexeren Modes paßt der Schrittmacher sich dabei auf physiologische Weise dem natürlichen Herzverhalten an.

Die Realisierung des dargestellten Konzepts kann gleichermaßen mittels diskreter oder programmierbarer Logik-Bauelemente erfolgen, wobei im Falle einer hardwaremäßigen Lösung die erforderlichen Verknüpfungen — wie dargestellt — mittels Logik-Gattern und Speichermitteln wie Flip-Flops und Latches erfolgen, während beispielsweise bei einer Mikroprozessorlösung die Logik-Entscheidungen nicht parallel, sondern nach Programm seriell vorgenommen werden. RAM-Speicherplätze übernehmen dabei die Funktion der Speichermittel zum Festhalten von für Betriebszustände charakteristischen Signalen. Einige der dargestellten Funktionsbaugruppen stellen vorteilhafte Weiterbildungen der Erfindung dar.

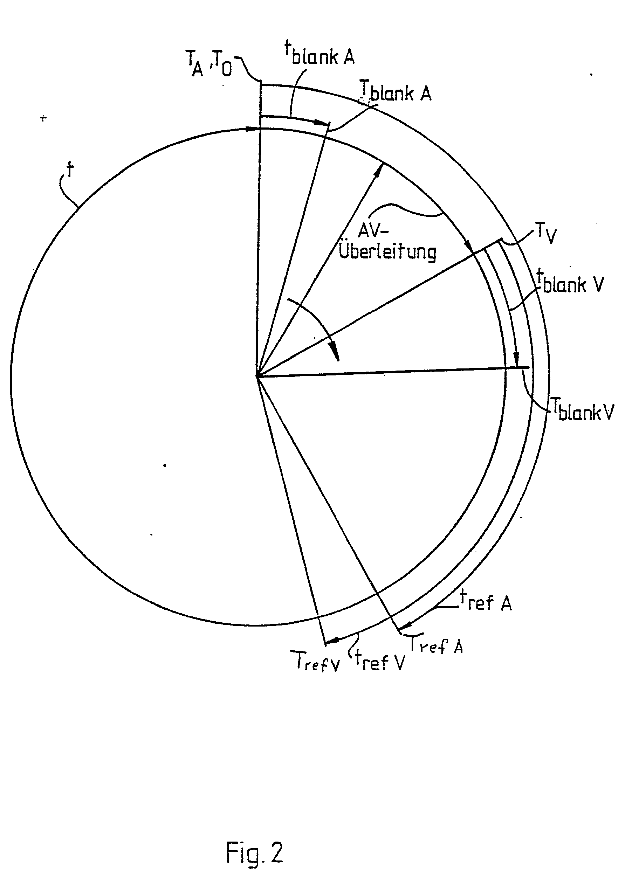

Aus Figur 2 ist der zeitliche Ablauf der Signalverarbeitung und Stimulationsvorgänge ersichtlich, wobei in den übrigen Darstellungen entsprechende Zeitbezeichnungen verwendet wurden, so daß diesbezüglich auf diese Figur stets Bezug genommen werden kann. In der Darstellung fällt der Anfangszeitpunkt $T_0$ zusammen mit dem Zeitpunkt $T_A$, der nach einem Umlauf (entsprechend einem Herzzyklus) erreicht wird und die Aktivität des Atriums charakterisiert. Im Falle einer Stimulation im Ventrikel oder Atrium folgt eine Austastzeit $t_{blankA}$ für die im Atrium plazierte Elektrode, wobei die Dauer dieser Austastzeit jeweils davon abhängig ist, ob im Atrium oder im Ventrikel, d. h. in derselben oder in der jeweils anderen Kammer stimuliert wurde. Das Ende dieser Austastzeit ist mit $T_{blankA}$ bezeichnet.

Zwischen $T_A$ und $T_V$ befindet sich die AV-Überleitungszeit, wobei $T_V$ den Zeitpunkt der Aktivität des Ventrikels charakterisiert. An $T_V$ schließt sich entsprechend eine Austastzeit $t_{blankV}$ an, welche im Zeitpunkt $T_{blankV}$ endet. Die Refraktärzeit des Atriums $t_{refA}$ beginnt mit $T_0$ und endet bei $T_{refA}$, während die Refraktärzeit des Ventrikels bei $T_V$ beginnt und bei $T_{refV}$ endet. Die Zeiten $T_{og}$ und $T_{tachy}$ sind aus dem Diagramm gemäß Figur 2 nicht direkt ersichtlich, da diese durch entsprechende Folgeraten der Herzaktionspotentiale und/oder Störsignale als Triggersignale festgelegt werden.

Bei dem in Figur 1 dargestellten Herzschrittmacher bilden die Anschlüsse A und V Ein- und Ausgänge für Signale, welche vom Herzen aufgenommen werden bzw. in Form von Stimulationsimpulsen an das Herz abgegeben werden. Mit « A » ist dabei der Anschluß für eine im Atrium und mit « V » der Anschluß für eine im Ventrikel fixierte Elektrode bezeichnet.

Die zentrale Steuerung für die zeitliche Zuordnung der abzugebenden Impulse erfolgt über einen Zähler 1, welcher durch von einem Taktgenerator 2 erzeugte Impulse inkrementiert wird und über einen « Reset »-Eingang in eine Ausgangsstellung zurücksetzbar ist. In Abhängigkeit von vom Herzen aufgenommenen Signalen ist dieser Zähler über einen zusätzlichen « Preset »-Eingang auf beliebige Zählerstände in Vorwärtsrichtung — entsprechend einer zeitlichen Voreilung — voreinstellbar. Ein Verändern des Zählerstandes in Rückwärtsrichtung ist nicht notwendig. Nach Erreichen seines Endstandes setzt der nachfolgende Impuls den Zähler auf 0, von wo aus der Zählvorgang fortgesetzt wird. Das Zusammenwirken des Zählers mit den übrigen in Figur 1 dargestellten Bauelementen wird weiter unten näher beschrieben. (Die Übertragung einzelner digitaler Signalimpulse oder analoger Signale ist in den Figuren durch einfache Verbindungen dargestellt, während doppelte Linien die Übertragung vollständiger Datenwörter symbolisieren.)

Die vom Atrium über eine dort fixierte Elektrode aufgenommenen Signale gelangen über den Anschluß A an eine Eingangsstufe 101, welche einen Vorverstärker enthält, der über einen Programmiereingang (zugehöriger Pfeil P) in seinem Verstärkungsfaktor einstellbar ist. Die Eingangsstufe bewirkt gleichzeitig eine Impulsformung in der Weise, daß die vom Atrium aufgenommenen Signale in Form von impulsförmigen Logiksignalen in den nachfolgenden Schaltstufen weiterverarbeitet werden können. Die Darstellung der Logikpegel bei dem wiedergegebenen Ausführungsbeispiel erfolgt in positiver Logik, d. h. das Vorhandensein eines Signals wird mit dem H-Zustand und die Abwesenheit mit dem L-Zustand bezeichnet. Umschaltelemente (Zähler, Mono- und Flip-Flops) werden durch die ansteigenden Vorderflanken der Signalimpulse aktiviert. Weiterhin weist die Eingangsstufe 101 Filtermittel auf, mit einer Filtercharakteristik, die sie vorzugsweise für die aufzunehmenden Nutzsignale durchlässig macht.

Die Eingangsstufe 101 wird außerdem von einer Austastschaltung 3 beeinflußt, welche die Eingangsstufe für vorgegebene Zeiträume $t_{blankA}$ sperrt, wenn ein Stimulationsimpuls an Atrium oder Ventrikel abgegeben wurde. Auf diese Weise wird verhindert, daß ein vom Schrittmacher abgegebener Stimulationsimpuls als herzeigenens Signal verarbeitet wird und das Betriebsverhalten des Schrittmachers fehlerhaft beeinflußt.

Der Eingangsstufe nachgeschaltet ist eine Störerkennungsschaltung 102, welche ein Ausgangssignal abgibt, wenn der Abstand zweier aufeinanderfolgender, von der Eingangsstufe 101 verarbeiteter Signalimpulse einen vorgegebenen zeitlichen Mindestabstand ($T_{og}$), entsprechend einer maximalen Folgefrequenz, unterschreitet. Derartige eine Störung bildende Signale können ihre Ursache sowohl außerhalb (elektromagnetische Einstreuungen oder Wechselströme) als auch innerhalb des Körpers des Patienten (Elektrodenartefakte) haben. Die Störerkennungsschaltung 102 verhindert im Zusammenwirken mit weiter unten zu beschreibenden Mitteln, daß an der Atriumelektrode aufgenommene Impulse, welche die vorgegebene Frequenz überschreiten, zur Weiterverarbeitung zugelassen werden.

Der Ausgang der Eingangsstufe 101 ist weiterhin mit dem Eingang einer Tachykardieerkennungsstufe 103 verbunden, welche entsprechend ein Signal abgibt, wenn die am Ausgang der Stufe 101 erscheinenden Impulse eine vorgegebene Folgezeit unterschreiten. Die « Tachykardierate » $T_{Tachy}$ ist mit ca. 250 ms größer als die als Kriterium für das Vorhandensein eines Störsignals festgelegte Zeitdauer $T_{og}$, welche ca. 100 ms beträgt.

Der Anschluß V, an den eine im Ventrikel zu fixierende Elektrode angeschlossen werden kann, ist mit dem Eingang einer weiteren Eingangsstufe 201 verbunden, die in ihren grundsätzlichen Funktionen der Eingangsstufe 101 für vom Atrium aufgenommene Signale entspricht. Der Verstärkungsfaktor ist über einen zusätzlichen Eingang (zugehöriger Pfeil P) einstellbar. Eine separate Störerkennungsschaltung 202 für die von der im Ventrikel fixierten Elektrode aufgenommenen Störsignale gibt ein Ausgangssignal ab, wenn die vom Ventrikel aufgenommenen Signalimpulse eine höhere als ein vorgegebene Frequenz aufweisen. Die Eingangsstufe 201 wird entsprechend durch von der Austaststufe 3 abgegebene Signale zeitweise gesperrt.

Stimulationsimpulse für das Atrium werden mittels einer Impulsformerschaltung 104 erzeugt, wobei die Amplitude der Stimulationsimpulse über einen zusätzlichen Eingang (zugehöriger Pfeil P) einstellbar ist. Die Impulsformerstufe 104 wird durch kurzzeitige Impulse mit logischem H-Pegel angesteuert und enthält auch die zur Heraufsetzung der Eingangsimpulse auf den zur Stimulation notwendigen Pegel erforderlichen Verstärkungsmittel.

Die von der Impulsformerstufe 104 abgegebenen Impulse gelangen einerseits zum Anschluß A und andererseits zu einer Run-away-Schutzschaltung 105, welche in dem dargestellten Ausführungsbeispiel durch ein Monoflop gebildet wird, dessen Impulsdauer auf die höchste zulässige Stimulationsrate im Atrium abgestimmt ist. Auf einen Ausgangsimpuls von der Impulsformerstufe 104 hin gibt die Schutzschaltung 105 einen Impuls vorgegebener Breite an den invertierenden Eingang eines UND-Gatters 106 ab, welches daraufhin für die Dauer des letztgenannten Impulses hin für an seinen anderen Eingang gelangende Signalimpulse gesperrt ist.

Eine Impulsformerstufe 204 für zum Stimulieren des Ventrikels bestimmte Impulse ist ebenfalls über einen weiteren Eingang (zugehöriger Pfeil P) bezüglich der Amplitude der abgegebenen Impulse beeinflußbar. Eine separate Run-away-Schutzschaltung 205 für den Ventrikel sperrt ein UND-Gatter 206 über dessen invertierenden Eingang, wenn die Folgezeit der den Ventrikel stimulierenden Impulse den vorgegebenen Wert unterschreitet.

Die Zeiten, zu denen Stimulationsimpulse abgegeben werden können (mit $T_A$ und $T_V$ bezeichnet), bestimmt der Zähler 1, wobei jeweils für Atrium und Ventrikel separate Vergleicherschaltungen 120 bzw. 220 vorgesehen sind, welche einen Impuls am Ausgang « = » erzeugen, wenn der Zählerstand des Zählers 1 mit einem vorgegebenen Zählerstand in einem Speicher 121 für den Stimulationszeitpunkt des Atriums bzw. einem entsprechenden Speicher 221 für den Ventrikel übereinstimmt. Die Vergleicherschaltung 220 gibt über einen zusätzlichen Ausgang « > » ein Signal ab, wenn der Zählerstand des Zählers größer ist als der in dem Speicher 221 festgehaltene Wert für $T_V$. Die genannten Zeitmarken $T_A$ und $T_V$ bilden Bezugszeiten für den Betrieb des Schrittmachers und beenden im Demand-Betrieb die sogenannten « Escape-Intervalle » innerhalb denen eine entsprechende Eigenaktion des Herzens die Erzeugung eines Stimulationsimpulses durch den Schrittmacher verhindert.

Die Zeiten $T_A$ und $T_V$ für Atrium und Ventrikel in den Speichern 121 bzw. 221 werden in Form von Zahlenwerten programmiert, welche diejenigen Zählerstände repräsentieren, die der Zähler 1 im Stimulationszeitpunkt erreicht haben muß. Die Zeitzuordnung zu den Zählerständen erfolgt in der Weise, daß die Stimulationszeitpunkte mit einem ausreichend feinen Zeitraster einstellbar sind. Bei einem Zählertakt von beispielsweise 1 kHz weist das zur Verfügung stehende Zeitraster eine Teilung in Millisekunden auf. Die mittels der Speicher 121 bzw. 221 vorgebbaren Zählerstände sind durch weiter unten darzustellende Mittel veränderbar.

Die Eingangsimpulse für die Ausgangsstufe 104, welche das UND-Gatter 106 passieren, stammen vom Ausgang eines ODER-Gatters 107, an dessen Eingänge alternativ die über die Abgabe eines Stimulationsimpulses an das Atrium entscheidenden Signale gelangen. Einer der Eingänge des ODER-Gatters 107 ist mit dem Ausgang eines UND-Gatters 108 verbunden, das ein Ausgangssignal abgibt, wenn die Vergleicherschaltung 120 das Erreichen des Zeitpunktes $T_A$ angibt und damit anzeigt, daß im Demand-Zustand der Vorhofstimulation ein Zeitpunkt erreicht wurde, bei dem wegen fehlender eigener Aktivität des Herzens ein Stimulationsimpuls erforderlich ist. Das UND-Gatter 108 wird über seinen weiteren Eingang mittels eines Signals durchgeschaltet, welches von einem Ausgang « time$_A$ » des Betriebszustandsregister 4 abgegeben wird, wenn eine Impulsabgabe nach Zeitablauf erwünscht ist. Bei freigegebener Signalaufnahme (sens$_V$ bei Block 4) für die entsprechende Kammer erfolgt damit ein « Demand »-Betrieb mit Inhibierung durch herzeigene Impulse, während bei gesperrter Signalaufnahme eine Stimulation zu festen Zeiten erfolgt.

Ein weiteres UND-Gatter 109 wird über einen entsprechenden Ausgang « trig$_A$ » der Schaltung 4 bei durch Herzeigenaktionen im Vorhof getriggerter Signalabgabe an den Vorhof aktiviert. Das Signal zum synchronen Auslösen des Stimulationsimpulses im Vorhof gelangt an den Eingang des UND-Gatters 109 vom Ausgang eines weiteren UND-Gatters 110, das seinerseits durch ein von einem Ausgang « sens$_A$ » der Schaltung 4 an seinen Eingang gelangendes Signal aktiviert wird, wenn die Aufnahme von Signalen aus dem Vorhof bei der gewählten Betriebsweise des Schrittmachers vorgesehen ist.

Es ist ersichtlich, daß für den durch herzeigene Signale getriggerten Betrieb diese Freigabe der Signalaufnahme Voraussetzung ist. An das UND-Gatter 110 gelangt nicht nur das Ausgangssignal der Eingangsstufe 101, sondern an dessen weiteren invertierenden Eingang auch das Ausgangssignal einer Refraktärstufe für den Vorhof 5, welche ein Signal abgibt, wenn für einen Zeitraum $t_{refA}$, der mit dem Erreichen des dem Zeitpunkt $T_A$ entsprechenden Zählerstand durch den Zähler 1 beginnt, die Verarbeitung der im Atrium aufgenommenen Signale zur Auswertung für die Abgabe von Stimulationsimpulsen gesperrt ist. Ein der Refraktärzeit $T_{refA}$ für das Atrium entsprechender Zahlenwert ist in einem Speicher 6 festgehalten und über weiter unten dargestellte Programmiermittel durch äußere Signale veränderbar.

Ein ODER-Gatter 207 und UND-Gatter 208 bis 210 bilden die entsprechenden, über die Abgabe von Stimulationsimpulsen an den Ventrikel bzw. über die Signalaufnahme aus dem Ventrikel entscheidenden Logik-Gatter. Das gewünschte Betriebsverhalten des Schrittmachers läßt sich ebenfalls über drei Ausgänge « sens$_V$ », « time$_V$ » und « trig$_V$ » der Schaltung 4 bestimmen, wobei das an den weiteren Eingang des UND-Gatters 208 gelangende Signal die Stimulation freigibt, wenn der Stand des Zählers 1 dem im Speicher 221 festgehaltenen, dem Zeitpunkt $T_V$ zugeordneten Zählerstand entspricht und somit zu diesem Zeitpunkt ein Ausgangssignal des Vergleichers 220 an das UND-Gatter 208 ausgegeben wird.

Die synchrone Stimulation wird durch das UND-Gatter 209 ausgelöst, dem das Ausgangssignal des UND-Gatters 210, welches die vom Ventrikel aufgenommenen Signalimpulse weiterleitet, zugeführt wird. Das UND-Gatter 210 ist über seinen invertierenden Eingang während der Ventrikel-Refraktärzeit $t_{refV}$ gesperrt, welche mittels eines Vergleichers 7 festgelegt wird, wobei der erreichte Zählwert des Zählers 1 mit dem in dem Speicher 8 festgehaltenen, die Zeitdauer $T_{refV}$ repräsentierenden Zählwert verglichen wird und zu dem Inhalt des Speichers 8 derjenige des Speichers 216, dessen Inhalt $T_V''$ im Normalfall der Zeit $T_V$ entspricht, hinzuaddiert wird. (Unterschiede zwischen der Funktion der Speicher 6 für die Refraktärzeit des Atriums und 7 für die Refraktärzeit des Ventrikels sind aus der folgenden Beschreibung ersichtlich). Der Inhalt des Speichers 8 ist wie derjenige des Speichers 6 über äußere Programmiermittel veränderbar.

Die Ausgangssignale der UND-Gatter 110 bzw. 210 beeinflussen des weiteren Schalter 122 und 222, mittels denen der Zähler 1 über seinen Preset-Eingang auf die in den Speichern 121 (für den Wert $T_A$) bzw. 221 (für den Wert $T_V$) vorhandenen Werte voreinstellbar ist. Damit wird der Zähler 1 beim Erscheinen eines intrakardialen Signals im Atrium auf der Zeit $T_A$ bzw. beim Erscheinen eines Signals im Ventrikel auf den der Zeit $T_V$ entsprechenden Zahlenwert vorangesetzt. Auf diese Weise wird der Schrittmacher mit dem Herzverhalten synchronisiert, wenn die UND-Gatter 110 bzw. 210 über ihre entsprechenden Signaleingänge für die Signalaufnahme vom Herzen freigegeben sind. Sind die zuletzt genannten Gatter gesperrt, so erfolgt die Stimulation gegebenenfalls asynchron.

Die Inhalte der Speicher 4, 6 und 8 sowie der eines weiteren Speichers 9, welcher die programmierbaren Verstärkungsfaktoren bzw. Ausgangssignalamplituden repräsentierenden Daten für die Stufen 101 und 201 sowie 104 und 204 (zugeordnete Pfeile P) enthält, sind über die blockschaltungsmäßig dargestellten Programmiermittel zu beeinflussen, welche nachfolgend kurz beschrieben werden sollen :

Die von außerhalb des Schrittmachers über einen geeigneten Nachrichtensignalträger (radiofrequente, optische oder sonstige Signale) in Form von Impulsen aufmodulierten, von einem entsprechenden — nicht dargestellten — Sender abgegebenen Signale, gelangen zu einem Empfänger 10, der die verstärkten Signale einem Dekoder 11 zuleitet, mit dessen Hilfe die zu übertragene ursprüngliche Programmierimpulsfolge wieder hergestellt wird. In einer Prüfstufe 12 wird mittels zusätzlich übertragener redundanter Signale festgestellt, ob die übertragene Information vollständig ist und letztere zutreffendenfalls an einen Steuerbaustein 13 übermittelt, durch den mittels einer ersten Teilinformation eine Auswahlschaltung 14 entsprechend aktiviert wird.

Durch das Ausgangssignal der Schaltung 14 wird eine der Schaltstufen 15 bis 20 betätigt, so daß die zweite übertragene Teilinformation in den mit der aktivierten Stufe der Stufen 15 bis 20 verbundenen Speicher gelangt und dort festgehalten wird. Die bereits erwähnten Speicherstufen 4, 6, 8, 9 sowie 121 und 222 halten die für den Betrieb des Schrittmachers wesentlichen veränderbaren Daten fest, wie sie in der vorangehenden Beschreibung aufgeführt wurden.

Der Speicher 4 ist von zusätzlichen Signalen beeinflußbar, welche aus der Signalverarbeitungsstufe 21 stammen, und eine Umschaltung der Betriebszustände in Abhängigkeit von vom Schrittmacher selbst aus dem Herzen aufgenommenen Signalen bewirkt, wobei die Eingangssignale der Stufe 21 von den Stufen 102 und 202 stammen und im Atrium bzw. im Ventrikel aufgenommene Störsignale repräsentieren, sowie von der Tachykardiestufe 103, welche ein Ausgangssignal abgibt, wenn der Tachykardiezustand festgestellt wurde.

Die von der strichpunktierten Linie umgebenen Bauelemente zur Signalverarbeitung und -speicherung sind jeweils nur zeitweise aktiviert, da die zum Betrieb des Schrittmachers notwendigen logischen Operationen mittels moderner mikroelektronischer Bauelemente in sehr kurzen Zeiten ausgeführt werden können, so daß deren permanenter Betrieb einen unnötigen Energieverbrauch zur Folge haben würde. Die außerhalb der genannten Linie befindlichen Bauelemente, die entweder direkt mit den im Herzen fixierten Elektroden verbunden sind und damit die Signalaufnahme vom Herzen kontrollieren, bzw. diejenigen Mittel, welche die Zeitgeber- und Zeitvergleichermittel bilden, sind dauernd aktiviert, da durch letztere bestimmt wird, zu welchen Zeitpunkten logische Operationen auszuführen sind.

Die Aktivierung der innerhalb der strichpunktierten Linie befindlichen Bauelemente erfolgt durch die von den Vergleichermitteln 120 bzw. 220 abgegebenen Signale, wenn ein vorgegebener Zeitpunkt erreicht wurde, oder aber durch die Ausgangssignale der Eingangsstufen 101 bzw. 201, wenn nämlich vom Herzen (außerhalb der jeweiligen Refraktärzeit) ein Signal aufgenommen wurde. Die Zusammenfassung der genannten Signale erfolgt über ein ODER-Gatter 30, welches über ein Monoflop 31 einen Schalter 32 betätigt, der die in der gewählten Darstellung innerhalb der strichpunktierten Linie angeordneten Bauelemente mit einer Energiequelle 33 verbindet, wobei dieses « Verbinden » mit einer Energiequelle in jedem Heraufsetzen der Energieversorgung, also auch in einem Umschalten der betreffenden Bauelemente von einem Bereitschafts-(Stand-by-) Zustand in einen Betriebszustand bestehen kann.

Die Impulsdauer des Monoflops 31 ist dabei so bemessen, daß zum Ausführen der notwendigen Schaltvorgänge ein ausreichend langer Zeitraum zur Verfügung steht. Diskrete Logikbauelemente lassen sich energiesparend bei derart herabgesetzter Versorgungsspannung betreiben, daß zwar keine Veränderungen vorgenommen werden können, gespeicherte Signalzustände jedoch erhalten bleiben. Diese Bauelemente dienen der nachfolgenden Signalverarbeitung in dem Sinne, daß sie festlegen, ob auf ein Eingangssignal oder beim Erreichen eines vorgegebenen Zeitpunkts ein Stimulationsimpuls abgegeben werden soll.

Der von der Energiequelle 33, die im dargestellten Ausführungsbeispiel von einer Batterie gebildet wird, ausgehende Pfeil E deutet an, daß die außerhalb der strichpunktierten Linie angeordneten Bauelemente direkt von der Energiequelle 33 ohne Zwischenschaltung des Schaltelementes 32 versorgt werden.

Mit der dargestellten Anordnung sind die Betriebsfunktionen üblicher Schrittmachertypen mit synchroner oder asynchroner Stimulation im Atrium und/oder Ventrikel erzeugbar, wobei je nach Zustand der Schaltmittel 4 und Freigabe der entsprechenden Stimulationssignale bzw. Signalaufnahmemöglichkeit über die UND-Gatter 108 bis 110 bzw. 208 bis 210 auch jene Schrittmacherbetriebsarten höherer Ordnung möglich sind, wie sie in Form einer Übersicht beispielsweise in dem Aufsatz « Physiological Cardiac Pacing », R. Sutton, J. Perrins und P. Citron in « PACE » ; Vol. 3, März-April 1980, S. 207 bis 219 wiedergegeben sind.

Das grundsätzliche Stimulationsverhalten der verschiedenen Schrittmacherbetriebsweisen bei verschiedenartigen Herzsignalen ist ebenfalls in der vorgenannten Literaturstelle angegeben.

Weitere Einzelheiten zu den in der nachfolgenden Beschreibung dargestellten Schaltungsteilen können den am gleichen Tage eingereichten, diese Schaltungsteile wegen der damit verbundenen Neuerungen näher beschreibenden Patentanmeldungen WO82/03780 bis 82/03785 derselben Anmelderin entnommen werden.

Durch die funktionsmäßige Trennung des komplexen, nur zeitweise aktivierten Logiksystems von den permanent betriebsbereiten Bauelementen, ist es in vorteilhafter Weise möglich, für den Fall des Ausfalls des umfangreichen Logikteils ein einfaches Ersatzsystem vorzusehen, durch welches ein Notbetrieb aufrecht erhalten werden kann. Obgleich zwar einerseits die Sicherheit einzelner Bauelemente gegen Ausfall in der Vergangenheit wesentlich gesteigert werden konnte, wird andererseits angestrebt, die Informationsverarbeitung in einem künstlichen Herzschrittmacher möglichst allen therapeutischen Anforderungen gerecht werden zu lassen, um dem Patienten eine optimale Stimulationstherapie zukommen zu lassen, und damit die erreichte Erhöhung der Zuverlässigkeit zum Teil wieder kompensiert.

Das dargestellte Konzept gestattet es, die Vorteile eines einfachen, höchst betriebssicheren Systems mit einem solchen höherer Verarbeitungsfähigkeit derart zu verbinden, daß bei Ausfall des komplexen Datenverarbeitungssystems ein einfaches Ersatzsystem bereitsteht, welches in der Lage ist, die Grundfunktionen des Schrittmachers unter nahezu allen Umständen für einen nur durch die Betriebsdauer der Energiequellen begrenzten Zeitraum sicherzustellen.

Diese Ersatzschaltung kann prinzipiell durch verschiedenartige Signale aktiviert werden. Eines dieser Signale ist ein von der komplexen Logikschaltung ausgehendes Signal « Par », das bei einer mittels eines Mikroprozessors realisierten Logikschaltung dann erscheint, wenn innerhalb der durchgeführten Operationen ein Plausibilitätsfehler ermittelt wurde, wie er beispielsweise durch eine Paritätsüberprüfung mit Hilfe von redundanten Signalen in bekannter Weise erzeugt werden kann. Entsprechende Signale lassen sich auch aus mit diskreten Logikbauelementen arbeitenden Schaltungen gewinnen, wenn hier zusätzliche Elemente verwendet werden, welche unzulässige Betriebszustände, beispielsweise in Form von Signalen, erkennen, die bei korrekt arbeitenden Logikmitteln beispielsweise nicht gleichzeitig oder aber nicht mit einer oberhalb oder unterhalb einer vorgegebenen Grenzfrequenz liegenden Rate etc. erscheinen dürfen. Derartige ein Fehlverhalten aufzeigenden Signale werden auch von den Ausgängen der Run-away-Schutzschaltungen 105 und 205 gewonnen und über ein ODER-Gatter 34 zusammengefaßt. Bei der dargestellten Anordnung kann es theoretisch in dem Fall zu einem Ansprechen der Run-away-Schutzschaltungen bei ordnungsgemäß arbeitenden Taktgenerator 2 kommen, wenn in die Speicher 121 oder 221 durch einen Fehler in dem durch die strichpunktierte Linie umrandeten Teil der Logikschaltungen fehlerhafte Zahlenwerte eingelesen wurden.

Dem ODER-Gatter 34 wird ein weiteres Signal M zugeführt, welches von einem Reed-Schalter stammt, der in der Zeichnung nicht dargestellt ist. Auf diese Weise ist es möglich, mittels eines von außen einwirkenden Magnetfeldes den Ersatzbetriebszustand entsprechend der « Magnetfrequenz » der bekannten Schrittmacher einzustellen.

Der Ausgang des ODER-Gatters 34 aktiviert eine Speicherschaltung 35, welche Zeitpunkten $T_A'$ und $T_V'$ entsprechende Zahlenwerte enthält, die bei Aktivierung dieser Speicherschaltung 35 mit Vorrang in die Speicher 121 bzw. 221 eingelesen werden. Gleichzeitig wird von der Schaltung 35 ein Ausgangssignal abgegeben, welches über invertierende Eingänge von UND-Gattern 111 und 211, die zwischen die Gatter 107 und 106 bzw. 207 und 206 eingeschaltet sind und diesen Signalweg sperren, während über die somit aktivierten UND-Gatter 112 und 212 eine direkte Verbindung zu den Ausgängen der Vergleicher 120 und 220 hergestellt wird.

Damit ist das im « D00 »-Betrieb arbeitende Ersatzsystem nicht mehr von der Signalverarbeitung der innerhalb der strichpunktierten Linie befindlichen Elemente der Schaltung abhängig. Die Werte $T_A'$ und $T_V'$ werden dabei so gewählt, daß sich eine für die Frequenzen annehmbare Ersatz-Stimulationsfrequenz ergibt. Die Betriebsart « D00 » bezieht sich auf den Anschluß zweier Elektroden. Ist jeweils nur eine der beiden Elektroden angeschlossen, so arbeitet der Schrittmacher im Zustand « A00 » oder « V00 » und gibt damit entsprechend der gewählten Elektrodenkonfiguration ebenfalls die zur Aufrechterhaltung der vitalen Funktionen notwendigen Stimulationsimpulse ab.

Mittels zweier in die Aktivierungsleitung des Schalters 222 eingefügter UND-Gatter 213 und 213a wird eine Synchronisation des Zählers 1 (und damit der Zeitsteuerung des Schrittmachers) auf vom Ventrikel erscheinende Signale für die Zeit der Überleitung vom Atrium zum Ventrikel verhindert (AV-Überleitung zwischen $T_A$ und $T_V$ in Figur 2), wenn gleichzeitig eine Signalaufnahme im Atrium möglich ist. Die entsprechenden Zeitmarken beziehen sich (abhängig von der Betriebsart des Schrittmachers) auf Zeiträume zwischen herzeigenen oder stimulierten Signalen. Dem UND-Gatter 213a werden das Signal $sens_A$ des Speichers 4 und das Signal «$>$» des Vergleichers 220 an seinen Eingängen zugeführt. Das Ausgangssignal des UND-Gatters 213a sperrt gegebenenfalls das UND-Gatter 213 über dessen invertierenden Eingang. Der weitere Eingang des UND-Gatters ist mit den die Signalaufnahme für das Atrium am UND-Gatter 110 freigebenden Signalausgang des Speichers 4 verbunden. Damit wird erreicht, daß eine Synchronisation bei regulärer Ventrikeltätigkeit stets nur mit $T_A$ erfolgt und somit das Atrium bei AV-sequentieller Stimulation die Führung behält bzw. bei fehlender Signalaufnahme aus dem Atrium der Abstand aufeinanderfolgender Zeitpunkte $T_A$ konstant bleibt.

Der hier beschriebene Schrittmacher stellt damit die physiologisch richtige Synchronisation mit dem Atrium sicher, wenn dort ungestörte Signale zur Verfügung stehen. Ist die Signalaufnahme im Atrium gestört, so erfolgt — entsprechend den vorgesehenen Möglichkeiten der Betriebszustandsänderungen, die weiter unten beschrieben werden — eine Umschaltung in einen eine Signalaufnahme im Ventrikel ermöglichenden Betriebszustand, so daß auch in diesem Fall eine korrekte Stimulation gewährleistet ist.

Obgleich bei freigegebener Signalaufnahme im Atrium für die Zeit t zwischen $T_A$ und $T_V$ keine Synchronisation des Zählers 1 durch im Ventrikel aufgenommene Signale erfolgt, wird die Refraktärzeit des Ventrikels dadurch in physiologisch sinnvoller Weise gesetzt, daß diese sich trotz fehlender Synchronisation des Zählers 1 auf die Zeiten bezieht, zu denen die Herzaktion im Ventrikel festgestellt wurde, was bei dem dargestellten Ausführungsbeispiel dadurch realisiert ist, daß das Ausgangssignal des UND-Gatters 210 über ein UND-Gatter 214 und ein Schaltelement 215 einen Speicher 216 mit einem zu diesem Zeitpunkt im Zähler 1 enthaltenen Zahlenwert lädt, so daß dieser Zeitpunkt $T_V''$ als Bezugszeitpunkt jetzt für die Ermittlung der Refraktärzeit $T_{refV}$ durch den Vergleicher 7 dienen kann. Erfolgt eine Signalaufnahme ausschließlich im Ventrikel, so ist eine Synchronisation des Zählers durch das Schaltelement 222 im Zeitpunkt $T_V$ freigegeben, wodurch mittels des UND-Gatters 214 in den Speicher 216 dann derjenige Zahlenwert für $T_V$ übertragen wird, welcher auch im Speicher 221 vorhanden ist.

Nach Ablauf der Refraktärzeit für aus dem Ventrikel aufgenommene Signale erscheinende Impulse, welche von Extrasystolen herrühren, synchronisieren den Zeitzähler 1 des Schrittmachers, was bei dem dargestellten Ausführungsbeispiel dadurch erreicht wird, daß der Zähler 1 durch die Schaltermittel 222 auf den Wert $T_V$, der im Speicher 221 vorhanden ist, gesetzt wird. Darüberhinaus muß auch sichergestellt sein, daß retrograd übergeleitete Erregungen nach Extrasystolen keine Synchronisation des Schrittmachers bewirken. Dies wird dadurch erreicht, daß nach Erkennen der Extrasystole (Ventrikelaktivität vor dem Zeitpunkt $T_0$) die Atriumrefraktärzeit $t_{refA}$ für einen Zyklus auf 400 ms gesetzt wird, so daß für einen ausreichend langen Zeitraum (entsprechend der retrograden Überleitungszeit) — ausgehend von der Zeitmarke $T_V$ das Atrium für eine Signalaufnahme gesperrt ist.

Die Sperrung derartiger retrograder Überleitungen ist deshalb von besonderer Bedeutung weil ein vom Schrittmacher auf das Signal im Atrium hin mit der vorgegebenen AV-Überleitungszeit abgegebener Stimulationsimpuls im Ventrikel eine Stimulation in der vulnerablen Phase zur Folge hätte.

Um hier den notwendigen Schutz zu bewirken, werden unmittelbar auf eine Extrasystole hin die Signalaufnahmemittel für Vorhof und Ventrikel für einen vorgegebenen Zeitraum refraktär geschaltet. Und zwar wird bei einem Eingangsimpuls am UND-Gatter 217 vom Ausgang der Eingangsstufe 201 für den Ventrikel, der erscheint, wenn auch der Vergleicher 220 am Ausgang «$>$» ein Signal abgibt, das an den zweiten Eingang des UND-Gatters 217 gelangt, ein Monoflop 218 gestartet. Dieses Monoflop gibt daraufhin einen Ausgangsimpuls von ca. 400 ms Dauer ab, welcher an einen Eingang des ODER-Gatters 219 gelangt, dessen zweiter Eingang mit dem Ausgang des Vergleichers 5 für die Refraktärzeit $T_{refA}$ verbunden ist, und dessen Ausgangssignal zu einem der Eingänge des UND-Gatters 110 übertragen wird.

Auf diese Weise ist sichergestellt, daß bei jeder nach der Zeit $T_V$ festgestellten Kammerkontraktion die Refraktärzeit für das Atrium neu gestartet und für die Zeitdauer des von dem Monoflop 218 abgegebenen Impulses (vorzugsweise 400 ms) aufrechterhalten wird, so daß eine Synchronisation des Schrittmachers durch retrograde Überleitungen verhindert ist. (Wie weiter unten näher erläutert ist, sind für diesen Zeitraum auch die Stör- und Tachykardieerkennungsmittel gesperrt, da diese bei einer retrograden Überleitung nicht ansprechen sollten. Die erforderliche Signalverbindung wird durch die Leitung vom Ausgang des Monoflops 218 zum Block 3 gebildet.) Ein ODER-Gatter sperrt auch den Ventrikel, wobei bevorzugt mittels — nicht dargestellter Schaltmittel — statt der Impulszeit des Monoflops 218 auch die in Block 7 enthaltene Zeit heranziehbar ist.

Für den Fall, daß gleichzeitig Signale im Atrium und Ventrikel aufgenommen werden, hat der Ventrikel Vorrang. Damit ist gewährleistet, daß die Sicherheit gegen schädliche Stimulation aufgrund unerwünschter Überleitungen auch bei nicht eindeutiger Signalerkennung zunächst einmal gegeben ist.

Gemäß einer bevorzugten Ausführung der Erfindung werden alle Umschaltungen (Programmänderungen, Betriebsartänderungen) zu Zeitpunkten vorgenommen, welche den Betriebsablauf nicht stören, das sind solche Zeitpunkte, zu denen keine Stimulation bewirkt werden kann und auch kein Eingangssignal erwartet wird, dessen Signalaufnahme wiederum von den eingestellten Betriebsparametern abhängig sein könnte. Ein derartiger Zeitpunkt ist derjenige auf $T_V$ folgende Zeitbereich, in dem beide Eingangsstufen refraktär sind (vergleiche Fig. 2, aus der die Zeiten des Herzzyklus im Hinblick auf den Betrieb des Schrittmachers in ihrer grundsätzlichen Verteilung ersichtlich sind).

Der mit $T_V$ beginnende Zeitraum ermöglicht dabei die für den Betrieb des Schrittmachers notwendigen Umschaltungen in einer Weise, welche die physiologischen Randbedingungen der Stimulation am wenigsten beeinträchtigen, da Umschaltungen zu dieser Zeit in eine natürliche ruheperiode des Herzens fallen. Im Zeitpunkt $T_V$ finden auch solche Überprüfungen statt, die regelmäßig durchgeführt werden müssen, wie die Bestimmung des Betriebszustands der Energiequelle (EOL-Block 36).

Der Zeitpunkt $T_V$ wird bei normalen Herzverhalten stets durchlaufen und wäre an sich ausreichend beispielsweise die Übernahme der geänderten Betriebsparameter (entsprechender Pfeil an Speicherblock 4) oder die Änderung der Programmierung aufgrund der von außen her übertragenen entsprechenden Signale zu diesem Zeitpunkt zu bewirken (Pfeil nach Block 13).

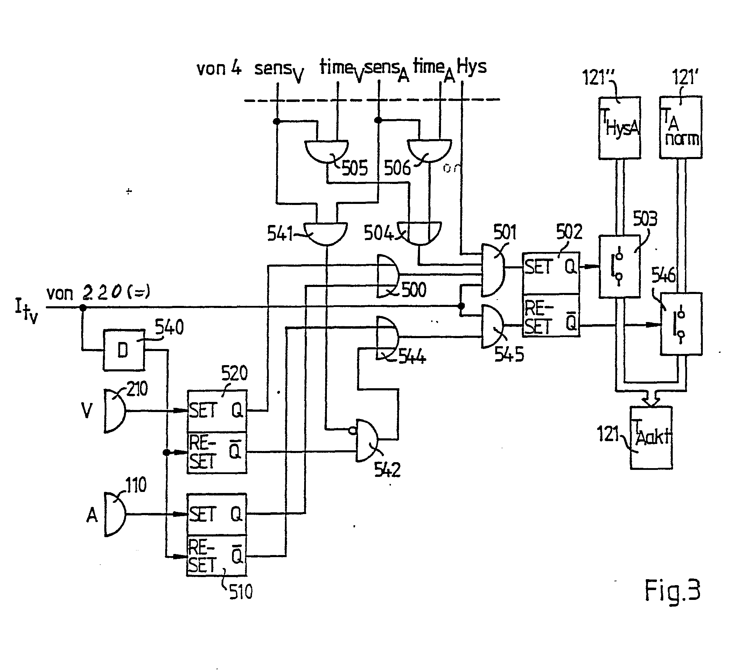

Die in Figur 3 dargestellte Schaltung stellt eine Zusatzschaltung dar, welche in Zusammenhang mit der Blockschaltung gemäß Fig. 1 den Betrieb des Schrittmachers mit Hysterese ermöglicht. Hysterese bedeutet in diesem Zusammenhang, daß bei aufgenommenen Herzeigenaktionen der Wert für den Zeitpunkt $T_A$ heraufgesetzt wird, so daß eine geringfügige Verringerung der Impulsrate der Herzeigenaktionen nicht zu Stimulationsimpulsen führt. Die Stimulation setzt erst dann wieder ein, wenn die vergrößerte Zeitdauer $T_{HysA}$ durch die Herzrate unterschritten wird.

Die in Figur 3 dargestellten Flip-Flops 510 und 520 werden jeweils gesetzt, wenn vom Ausgang des in Figur 1 dargestellten UND-Gatters 110 bzw. 210 ein Signal erscheint, d. h. im Atrium oder Ventrikel eine Herzeigenaktion aufgenommen wurde. Beide Ereignisse führen über ein ODER-Gatter 500 und ein UND-Gatter 501 im Zeitpunkt $T_V$ zum Setzen eines den Hysteresezustand speichernden Flip-Flops 502. Mittels dieses Flip-Flops 502 wird in den Speicher 121 anstelle des Normalwertes für die Impulsrate des Atriums $T_A$ der in einem Speicher 121′ festgehalten ist und durch Programmierung in den Schrittmacher übertragen wurde, mittels des Schaltelementes 503 ein Wert $T_{HysA}$ aus einem Speicher 121″ überführt und den folgenden Datenvergleichs- und Entscheidungsoperationen zugrundegelegt.

Ein Setzen des Flip-Flops 502 kann dabei nur dann erfolgen, wenn die weiteren Eingänge des UND-Gatters 501 den logischen H-Zustand einnehmen, d. h. über den Eingang Hys (durch Setzen eines entsprechenden Bits im Programmierspeicher 4) der Hysterese-Zustand freigegeben wurde und entweder im Atrium oder im Ventrikel jeweils sowohl eine Signalaufnahme vom Herzen als auch eine Stimulationsimpulsabgabe nach Zeitablauf erfolgen kann, wie es durch die dargestellte Verknüpfung eines ODER-Gatters 504 mit zwei UND-Gattern 505 und 506, denen die vorgenannten Eingangsbedingungen in Form von logischen Signalen von Speicher 4 (Fig. 1) zugeführt sind, realisiert ist.

Die Flip-Flops 510 und 520 werden jeweils kurzzeitig, nachdem ihr Zustand in das Flip-Flop 502 übertragen wurde, durch den um einen Zeitraum mittels eines Verzögerungsgliedes 540 verzögerten Impuls $T_V$ zurückgesetzt, so daß für jeden Herzzyklus wieder neu über das Vorliegen der Bedingungen für die Hysterese entschieden werden muß. Wird bis zum Zeitpunkt $T_V$ keine Herzaktion in der Kammer, von der Signale aufgenommen werden, erkannt, so wird — wie nachfolgend dargestellt — der nicht gesetzte Zustand der Flip-Flops 510 bzw. 520 in das Flip-Flop 502 übertragen. Damit wird bei Betriebsarten, welche eine ausschließliche Signalaufnahme entweder im Atrium oder im Ventrikel gestatten, durch diese Signale für sich der Hysteresezustand jeweils gesetzt oder zurückgesetzt werden.

Wenn eine Signalaufnahme im Atrium und im Ventrikel möglich ist (beispielsweise Betriebsart VDT/I oder DDD), soll der Hysteresezustand nicht schon dann zurückgesetzt werden, wenn nur die AV-Überleitung ausfällt, der normale Sinusrhythmus jedoch aufrechterhalten bleibt, da die nachfolgende Stimulation im Ventrikel anschließend im Zeitablauf erfolgen kann, ohne daß eine Änderung der atrialen Rate erforderlich ist. Durch die weiteren Elemente der in Fig. 3 dargestellten Schaltung wird verhindert, daß der Hysteresezustand allein deswegen zurückgesetzt wird, weil keine AV-Überleitung stattfindet, obgleich spontane Atriumaktionen vorhanden sind und nur eine Stimulation im Ventrikel notwendig ist.

Ein Zurücksetzen des Flip-Flops 510 wird aber dann nicht auf das Flip-Flop 502 übertragen, wenn zwei Bedingungen erfüllt sind:

1. Es ist eine Signalaufnahme vom Atrium als auch eine solche vom Ventrikel möglich.

2. Es wird nur im Atrium ein zu einer Herzeigenaktion gehöriger Impuls aufgenommen — es fehlt also lediglich die aufgrund einer physiologischen AV-Überleitung hervorgerufene Ventrikelkontraktion.

Eine Atriumstimulation bewirkt hingegen ein Zurücksetzen.

Die erste Bedingung ist durch Verknüpfung der entsprechenden Eingangssignale von dem Speicher 4 in Fig. 1 über ein UND-Gatter 541 realisiert, dessen Ausgangssignal dem invertierenden Eingang eines UND-Gatters 542 zugeführt ist. Der weitere Eingang des UND-Gatters 542 ist mit dem invertierenden Ausgang $\bar{Q}$ des Flip-Flops 520 verbunden. Das Ausgangssignal des UND-Gatters 542 und das invertierte Ausgangssignal $\bar{Q}$ des Flip-Flops 510 gelangen an Eingänge eines ODER-Gatters 544, dessen Ausgangssignal mit dem Signalimpuls $I_{TV}$ mittels eines UND-Gatters 545 so verknüpft ist, daß entsprechend den vorgenannten Bedingungen beim Fehlen eines herzeigenen Signals im Atrium oder Ventrikel der zurückgesetzte Zustand des Flip-Flops 520 in das Flip-Flop 502 übernommen wird, wenn eine Signalaufnahme nur dort möglich ist. Anschließend wird durch den Schalter 546 wieder der ursprüngliche in dem Speicher 121' gespeicherte Zahlenwert $T_{Anorm}$ in den Speicher 121 als aktueller Wert $T_{Aakt}$ übertragen. Wie weiter unten dargestellt ist, wird auch die Refraktärzeit des Ventrikels jeweils der aktuellen Atriumrate $T_{Aakt}$ angepaßt.

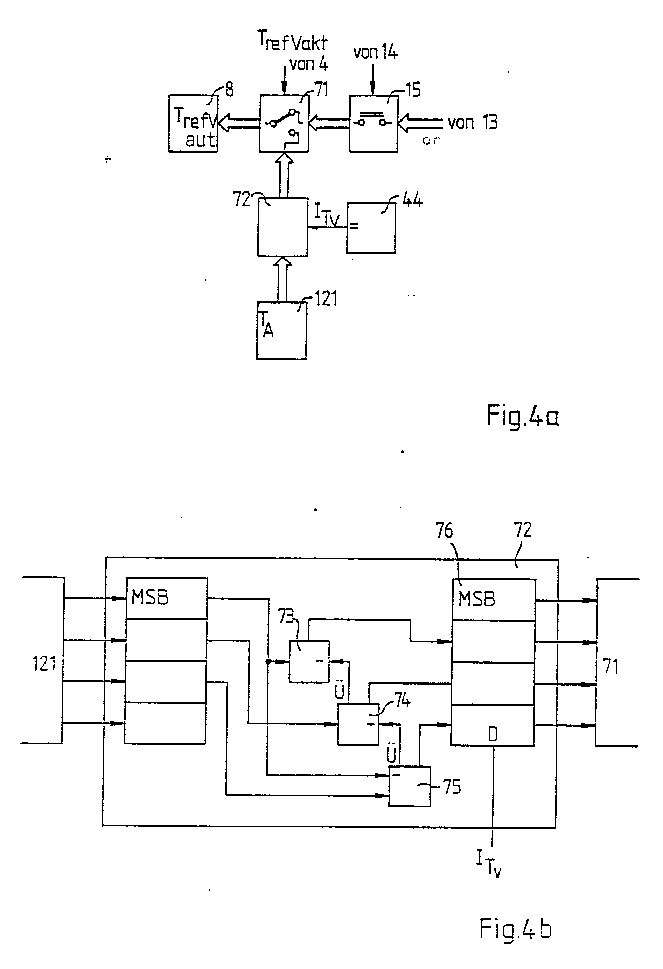

In Figur 4a ist eine Anordnung blockschaltungsmäßig dargestellt, welche eine automatische Anpassung der Refraktärzeit des Ventrikels an die programmierte Herzrate bewirkt.

Da bei programmierbaren Schrittmachern bei im wesentlichen fester AV-Überleitungszeit mit sich verändernder Herzrate (Folge der Impulse $T_A$) sich bei fester Refraktärzeit für den Ventrikel der (ungeschützte) Zeitraum bis zum nächsten $T_A$-Impuls Schwankungen unterliegen würde, die genauso groß ist, wie die auftretende Differenz bei der Änderung der gesamten Folgezeiten der Impulse im Atrium entsprechend der gewählten Rate, ist es günstig, die Refraktärzeit des Ventrikel entsprechend proportional zu variieren, um einerseits eine Stimulation zu ungünstigen Zeitpunkten zu vermeiden — andererseits aber ein möglichst großes Zeitfenster zur Verfügung zu haben, in dem eine Signalaufnahme vom Ventrikel möglich ist.

Zusätzlich zu den Baugruppen gemäß Fig. 1 ist zwischen den Speicher 8 und den Schalter 15 ein Umschalter 71 eingefügt, welcher durch ein Signal $T_{refVaut}$ ansteuerbar ist, das durch eine entsprechende Programmierung als Ausgangssignal der Baugruppe 4 in Fig. 1 erzeugbar ist und anzeigt, daß die Berechnung der Refraktärzeit für den Ventrikel automatisch erfolgen soll. In durch das vorgenannte Signal aktiviertem Zustand verbindet der Umschalter 71 den Speicher für $T_{refV}$ mit einer Rechenschaltung 72, welche aktiviert durch den Vergleicher 220 bei Erscheinen des Signals « = » aufgrund der im Speicher 121 enthaltenen Größe für die Atriumrate $T_A$ einen Rechenvorgang ausführt und die ermittelten Daten für die Refraktärzeit des Ventrikels in einem dafür vorgesehenen Speicher festhält.

Einzelheiten der Rechenschaltung 72 gehen aus Fig. 4b hervor, welche den Aufbau dieser Schaltung am Beispiel von jeweils vier Bit enthaltenen Datenworten darstellt. Die Berechnung von $T_{refVaut}$ erfolgt nach der Formel :

$$T_{refVaut} = (1/2 - 1/8) \cdot T_A$$

Diese Rechnung läßt sich in besonders einfacher Form binär durchführen, wobei die Eingangssignale vom wichtigsten (MSB) Bit her in der angegebenen Reihenfolge drei Subtrahierstufen für einzelne Bits zugeführt werden, die ein Ausgangssignal und ein Übertragungssignal abgeben. Die Division durch zwei entspricht einer Verschiebung um eine Stelle zu niedrigeren Bit-Werten hin, während die Division durch acht eine Verschiebung um drei Stellen ausmacht. Ein Rest bei der Division wird nicht berücksichtigt. Die derart in ihrer Position verschobenen Daten werden in den einzelnen Subtrahierstufen 73 bis 75 voneinander abgezogen und die Ergebnisse den Speicherzellen eines 4-Bit-Speichers 76 zugeführt, welcher die Daten jeweils auf einen Impuls zum Zeitpunkt $T_V$ hin festhält. Das Ausgangssignal $\ddot{U}$ der höchstwertigen Subtrahierstufe 72 gelangt dabei in die zweithöchstwertige Stufe des Speichers 76, während die anderen Bits entsprechend ihrer Wertigkeit folgen. Der Berechnungsvorgang wird immer dann ausgeführt, wenn sich der für $T_A$ festgehaltene und für die Steuerung des Schrittmachers maßgebliche Wert ändert, was bei der dargestellten Ausführungsform jeweils im Zusammenhang mit dem Zeitpunkt $T_V$ erfolgt.

### Patentansprüche

1. Herzschrittmacher mit Mitteln zur Sperrung von im Atrium bzw. im Ventrikel aufgenommenen Signalen für die Auswertung zur Abgabe von Stimulationsimpulsen (Refraktärzeit) und einem ersten veränderbaren Speicher zum Festhalten eines einen maximalen Zeitabstand $T_A$ zwischen aufeinanderfolgenen Impulsen im Atrium ohne Auslösung einer Stimulation definierenden Wertes, gekennzeichnet durch Schaltmittel, die den Inhalt eines zweiten Speichers (8) für die Festlegung der Refraktärzeit des Ventrikels in Abhängigkeit vom Inhalt des ersten Speichers (121) derart beeinflussen, daß mit zunehmendem Zeitabstand die Dauer der Refraktärzeit heraufgesetzt bzw. mit abnehmendem Zeitabstand verringert wird.

2. Herzschrittmacher nach Anspruch 1, dadurch gekennzeichnet, daß die Veränderung der Refraktärzeit proportional mit der Veränderung des Zeitabstands erfolgt.

3. Herzschrittmacher nach Anspruch 2, dadurch gekennzeichnet, daß die Veränderungen der Refraktärzeit $T_{refVaut}$ nach der Formel

$$T_{refVaut} = (1/2 - 1/8) \cdot T_A$$

erfolgt.

4. Herzschrittmacher nach einem der vorangehenden Ansprüche, dadurch gekennzeichnet, daß die Schaltmittel zur Veränderung der Refraktärzeit durch die Änderung des Zeitabstands bei Hysteresebetrieb beeinflußt werden.

5. Herzschrittmacher nach einem der vorangehenden Ansprüche, dadurch gekennzeichnet, daß auch während der Refraktärzeiten in Ventrikel und/oder Atrium aufgenommene Signale Schaltmitteln zur Erkennung von Stör- und/oder Tachykardiezuständen (102, 202, 103) zugeleitet werden.

## Claims

1. A cardiac pacemaker having means for blocking signals picked up in the atrium or in the ventricle for evaluation for the delivery of stimulation pulses (refractory time) and a first variable store for retaining a value defining a maximum interval of time $T_A$ between successive pulses in the atrium without triggering a stimulation, characterised by circuit elements which influence the contents of a second store (8) for retaining the refractory time of the ventricle depending on the contents of the first store (121) in such a manner that as the interval of time increases, the duration on the refractory time is increased and as the interval of time decreases, it is reduced.

2. A cardiac pacemaker as claimed in Claim 1, wherein the variation in the refractory time is effected in proportion to the variation in the interval of time.

3. A cardiac pacemaker as claimed in Claim 2, wherein the variations in the refractory time $T_{refVaut}$ are effected in accordance with the formula

$$T_{refVaut} = (1/2 - 1/8) \cdot T_A$$

4. A cardiac pacemaker as claimed in one of the preceding Claims, wherein the circuit elements for varying the refractory time are influenced by the variation in the interval of time during hysteresis operation.

5. A cardiac pacemaker as claimed in one of the preceding Claims, wherein signals picked up in ventricle and/or atrium even during the refractory times are fed to circuit elements for detecting states of disturbance and/or of tachycardia (102, 202, 103).

## Revendications

1. Stimulateur cardiaque comportant des moyens pour bloquer des signaux recueillis dans l'oreillette ou le ventricule en vue de l'exploitation pour la délivrance d'impulsions de stimulation (période réfractaire), ainsi qu'une première mémoire modifiable pour retenir une valeur définissant un intervalle de temps maximal $T_A$ entre des impulsions successives dans l'oreillette sans déclenchement d'une stimulation, caractérisé par des moyens de commutation qui influencent le contenu d'une seconde mémoire (8), pour la fixation de la période réfractaire du ventricule en fonction du contenu de la première mémoire (121), de manière que la durée de la période réfractaire soit accrue à mesure que l'intervalle de temps augmente, et de manière que la durée de la période réfractaire soit réduite à mesure que l'intervalle de temps diminue.

2. Stimulateur selon la revendication 1, caractérisé en ce que le changement de la période réfractaire s'effectue proportionnellement au changement de l'intervalle de temps.

3. Stimulateur selon la revendication 2, caractérisé en ce que les changements de la période réfractaire $T_{refVaut}$ s'effectuent selon la formule

$$T_{refVaut} = (1/2 - 1/8) \cdot T_A$$

4. Stimulateur selon une des revendications précédentes, caractérisé en ce que les moyens de commutation pour changer la période réfractaire sont influencés par le changement de l'intervalle de temps en cas de fonctionnement à hystérésis.

5. Stimulateur selon une des revendications précédentes, caractérisé en ce que des signaux recueillis pendant les périodes réfractaires, dans le ventricule et/ou l'oreillette, sont appliqués également à des moyens de commutation (102, 202, 103) pour la détection d'états de perturbation et/ou de tachycardie.

Fig. 1

Fig. 2

0 077 809

Fig.3

3

**0 077 809**

Fig.4a

Fig.4b

4